# EUROPÄISCHE PATENTSCHRIFT

(11) **EP 0 716 304 B1**
(45) Veröffentlichungstag und Bekanntmachung des Hinweises auf die Patenterteilung: **04.10.2001**
(21) Anmeldenummer: 95117768.2
(22) Anmeldetag: 11.11.1995
(51) Int. Cl.: G01N 33/531, G01N 33/533, G01N 33/53, G01N 33/577, G01N 33/58

(54) **Immunoassays für Haptene und hierfür verwendbare Haptentracer-Antikörper-Komplexe sowie Verfahren zur Herstellung derselben**
Immunoassays for haptens and hapten-tracer-antibody complexes to be used therein and also method to produce them
Essais immunologiques pour des haptènes et des complexes d'haptènes-traceurs-d'anticorps pour être utilisé à cet égard et aussi méthode de leur production

(30) Priorität: 10.12.1994 DE 4444002
(43) Veröffentlichungstag der Anmeldung: 12.06.1996
(73) Patentinhaber: Dade Behring Marburg GmbH, 35001 Marburg (DE)
(72) Erfinder: Neuenhofer, Stephan, Dr., D-35037 Marburg (DE); Käsmarker, Reinhard, D-35041 Marburg (DE)

(56) Entgegenhaltungen:
- EP-A- 0 135 071
- EP-A- 0 442 372

## Beschreibung

Die vorliegende Erfindung betrifft das Gebiet der Immunoassays. Häufig ist es wünschenswert nachzuweisen, ob ein bestimmtes Antigen, d.h. eine Verbindung, die eine immunologische Reaktion hervorrufen kann, in einer Probe vorliegt oder nicht. Der Nachweis des Antigens wird oft durch sogenannte Immunoassays geführt, wobei mit Hilfe von immunologischen Methoden das Antigen oder das Hapten nachgewiesen wird. Die vorliegende Erfindung betrifft insbesondere Immunoassays, bei denen sogenannte Haptentracer-Komplexe eingesetzt werden. Bei den Haptentracem ist das immunologisch relevante Hapten mit einer Anzeigekomponente verbunden.

Aus dem Stand der Technik sind chemolumineszent markierte Haptenkonjugate bekannt. In der EP 0 135 071 werden chemolumineszent markierte Haptenkonjugate beschrieben, welche zwischen der zur Chemolumineszenz befähigten Gruppe und dem Hapten eine Verbindungsgruppe, die auch "Spacer" genannt wird, aufweisen. Bei der Verknüpfungsgruppe handelt es sich um ein kettenförmiges Polymer mit wiederkehrenden funktionellen Gruppen wie Peptide, Glykoproteine, Glykolipide oder Kohlenhydrate.

In der EP 0 442 372 werden markierte Haptene beschrieben, bei denen eine als Label bezeichnete Anzeigekomponente mit dem Hapten über ein Polyethylenglykol und ein Protein bzw. Oligo- oder Polypeptid verknüpft ist. Die Anzeigekomponente ist eine mit chemischen oder physikalischen Methoden quantifizierbare Gruppe.

Es wurde festgestellt, daß die mit einer Anzeigekomponente verbundenen Haptene in der Praxis gewisse Nachteile aufweisen. So sind diese Haptenkonjugate in der Regel nicht lagerstabil, insbesondere, wenn es sich um wäßrige Lösungen handelt, und die Lagerung nicht im tiefgefrorenen Zustand erfolgt, da die Gefahr einer Hydrolyse besteht. Weiterhin wurde festgestellt, daß derartige Haptenkonjugate aufgrund von unspezifischen Bindungen häufig nicht reproduzierbare Ergebnisse liefern. Zwar kann in manchen Fällen durch entsprechende Zusatzstoffe, wie beispielsweise Proteine, die unspezifische Bindung von Haptentracern an Gefäßwände reduziert werden, jedoch beeinträchtigen diese Zusatzstoffe nicht selten die Qualität des Tests.

Im praktischen Einsatz kann es auch weitere Probleme dadurch geben, daß der Anwender der Testlösungen mehrere Tracerlösungen für einen größeren Ansatz poolt. Durch die Verwendung eines weiteren Gefäßes treten dann erneut unkontrollierte Adsorptionsphänomene auf, deren Ausmaß von Art (Glas, Polyethylen, Polystyrol etc.) und Größe des verwendeten Gefäßes sowie von der Temperatur abhängen kann. Dadurch kann die Eignung des Haptentracers im Testassay nicht mehr garantiert werden.

Gegenstand der vorliegenden Erfindung sind daher Haptentracer-Komplexe, die dadurch gekennzeichnet sind, daß sie einerseits ein Hapten aufweisen, das direkt oder über eine Abstandsgruppe mit einer Anzeigekomponente verbunden ist und andererseits einen Antikörper aufweist, der spezifisch an die Anzeigekomponente binden kann.

Bei einer Anzeigekomponente handelt es sich um eine signalgebende Komponente, die auch als Label oder Marker bezeichnet werden kann.

Die erfindungsgemäßen Haptentracer-Komplexe bestehen einerseits aus einem Hapten, das direkt oder über eine Abstandsgruppe mit einer Anzeigekomponente verbunden ist. Hapten ist eine Bezeichnung für ein Antigen, das aufgrund seiner Größe in der Regel erst nach Kopplung an einen Träger die Bildung von Antikörpern induzieren kann. Die Anzeigekomponente ist eine Gruppe, die mit chemischen oder physikalischen Methoden nachweisbar bzw. quantifizierbar ist. Hapten und Anzeigekomponente können entweder direkt über eine chemische Bindung gebunden sein oder zwischen den beiden Komponenten kann sich eine Abstandsgruppe, die auch als Spacer bezeichnet wird, befinden. Üblicherweise werden als Abstandsgruppe lineare Strukturen verwendet wie beispielsweise Polyethylenglykol. Es.ist aber auch durchaus möglich, als Abstandsgruppe andere kettenförmige Polymere mit wiederkehrenden funktionellen Gruppen wie Peptide, Glykoproteine, Glykolipide oder Kohlenhydrate zu verwenden. Typische Beispiele für derartige Abstandsgruppen sind Polysaccharide wie Dextrane, Pectine, Lectine, Peptide wie Polylysin, Proteine wie Serum-Albumin oder Globuline, oder Glykoproteine wie Transferin.

Die erfindungsgemäßen Haptentracer-Komplexe weisen weiterhin einen Antikörper auf, der spezifisch an die Anzeigekomponente binden kann. Derartige Antikörper, die gegen die Anzeigekomponente spezifisch sind, werden nach an sich bekannten immunologischen Verfahren erhalten. Polyklonale Antikörper, die gegen die Anzeigekomponente gerichtet sind, werden dadurch erhalten, daß Labortiere, beispielsweise Kaninchen, mit der Anzeigekomponente (vorzugsweise einem Label-Protein-Konjugat) immunisiert werden. Alternativ hierzu können monoklonale Antikörper nach dem bekannten Verfahren von Köhler und Milstein hergestellt werden, die spezifisch gegen die Anzeigekomponente gerichtet sind.

Die erfindungsgemäßen Haptentracer-Komplexe können grundsätzlich bei allen entsprechenden Immunoassays, vorteilhafterweise bei heterogenen Immunoassays, verwendet werden. Bei diesen Tests wird ein Antikörper, der spezifisch ist für das nachzuweisende Antigen bzw. Hapten, an eine feste Phase entweder über eine kovalente Bindung oder durch Adsorption gekoppelt. Dieser Antikörper, der für das Hapten spezifisch ist, wird in bevorzugter Weise dadurch gewonnen, daß dieses Hapten an ein Polymer, beispielsweise an Serum-Albumine, kovalent gekoppelt wird und das zur Antikörper-Herstellung ausgewählte Tier wird damit immunisiert. Es hat sich gezeigt, daß derartige Antikörper hochspezifisch sind sowohl für das reine Hapten wie auch für das mit einer Anzeigekomponente verbundene Hapten. Als Haptene kommen alle analytisch interessanten organischen Substanzen in Frage, die eine immunologische Reaktion in einem Gasttier hervorrufen können, wenn sie in Form eines immunogenen Konjugats aus dem Hapten und einem Trägermolekül zur Immunisierung injiziert werden.

Der für das Hapten spezifische Antikörper wird in der Regel an einen festen Träger gebunden. Beispiele für derartige Träger sind Polystyrolröhrchen oder kügelchen, an die der Antikörper durch Adsorption fixiert wird, oder mit Lysinhaltigen Polypeptiden vorbehandelte Kunststoffoberflächen, an die der Antikörper über bifunktionelle Reagenzien kovalent gebunden wird oder mit NH₂-Gruppen oder COOH-Gruppen funktionalisierte magnetische Latex-partikel, an die der Antikörper beispielsweise mit Hilfe von Carbodiimiden gebunden wird.

In einer bevorzugten Ausführungsform der vorliegenden Erfindung ist die Anzeigekomponente eine zur Chemolumineszenz fähige Gruppe, wobei diese Gruppe besonders bevorzugt ausgewählt ist aus der Gruppe umfassend Acridiniumacylsulfonamid, Acridiniumester, Luminol, Isoluminol, Dioxetan, Oxalsäurester oder Oxalsäureamid oder ein Derivat einer dieser Verbindungen.

Unter Chemolumineszenz wird die Emission von Licht bei einer chemischen Reaktion verstanden. Im Rahmen der vorliegenden Erfindung werden daher solche Verbindungen an das Hapten gebunden, die zu chemolumineszenten Reaktionen fähig sind. Hierbei kann es sich um einen Acridiniumester, Acridiniumacylsulfonamid, Luminol oder dessen Derivate, ein Isoluminol oder dessen Derivate, ein Dioxetan, ein Luciferin, ein Oxalsäureester oder ein Oxalsäureamid handeln.

In einer anderen bevorzugten Ausführungsform ist die Anzeigekomponente eine zur Fluoreszenz befähigte Gruppe. Hierbei wird als Anzeigekomponente ein Fluoreszenzfarbstoff verwendet. Ein Beispiel für einen derartigen Fluoreszenzfarbstoff ist das Fluoresceinisothiocyanat.

In einer anderen bevorzugten Ausführungsform ist die Anzeigekomponente eine zur Biolumineszenz fähige Gruppe. Bei der Biolumineszenz handelt es sich um eine Form der Lumineszenz, die bei niederen Organismen wie Bakterien, Pilzen, Hohltieren, Würmern, Krebsen usw. auftritt. Bei der Biolumineszenz wird allgemein Luciferin in Gegenwart von Sauerstoff unter ATP-Verbrauch durch Luciferasen enzymatisch in aktiviertes Oxiluciferin umgewandelt, das unter Lichtabgabe (sichtbares blaues Licht) Oxiluciferin bildet.

In einer anderen Ausführungsform der vorliegenden Erfindung kann die Anzeigekomponente eine Gruppe sein, die zur Elektrolumineszenz fähig ist. Die Elektrolumineszenz wird durch elektrische Feldstärken erzielt.

In einer weiteren Ausführungsform der vorliegenden Erfindung ist die Anzeigekomponente zur Phosphoreszenz fähig. Bei der Phosphoreszenz geben elektronisch angeregte anorganische oder organische Verbindungen frühestens 10⁻³ Sekunden nach der Anregung die absorbierte Energie in Form von Strahlung meist längerer Wellenlänge wieder ab.

Im Rahmen der vorliegenden Erfindung können die gegen die Anzeigekomponente gerichteten Antikörper monoklonale Antikörper, polyklonale Antikörper, Antikörperfragmente, chemisch modifizierte Antikörper oder chemisch modifizierte Antikörperfragmente sein.

Die erfindungsgemäßen Haptentracer-Komplexe können auf einfache Art und Weise hergestellt werden. In bevorzugter Weise werden die erfindungsgemäßen Haptentracer-Antikörper-Komplexe dadurch hergestellt, daß man den gegen die Anzeigekomponente gerichteten Antikörper mit dem markierten Haptenderivat in wäßriger Lösung zusammenbringt. Die wäßrige Reaktionslösung kann gegebenenfalls zur Löslichkeitsverbesserung des Haptentracers organische Lösungsmittel in Konzentrationen von größer 0 bis etwa 50 % enthalten. Bevorzugt eingesetzt werden hierbei Acetonitril, Dimethylformamid oder Dimethylsulfoxid.

Gegenstand der vorliegenden Erfindung sind auch Testkits, die zur Durchführung von Immunoassays zum Nachweis eines Antigens bzw. Haptens geeignet sind. Wesentlich ist, daß hierbei wenigstens ein erfindungsgemäßer Haptentracer-Antikörper-Komplex eingesetzt wird.

Unter Testkits im Sinne der vorliegenden Erfindung werden diejenigen Sachgesamtheiten verstanden, die zur Durchführung eines Immunoassays erforderlich sind. In der Regel umfassen die Testkits einen Gegenstand, der eine feste Phase zur Verfügung stellt, wie Mikrotiterplatten, Polystyrolröhrchen oder ähnliches, wenigstens einen Antikörper, der gegen das Hapten gerichtet ist und wenigstens einen erfindungsgemäßen Haptentracer-Antikörper-Komplex.

In einer bevorzugten Ausführungsform umfassen die erfindungsgemäßen Testkits weiterhin diejenigen Komponenten, die zur Durchführung eines Lumineszenzimmunoassays erforderlich sind.

In einer besonders bevorzugten Ausführungsform umfassen die erfindungsgemäßen Testkits auch magnetisierbare Partikel. Derartige Immunoassays werden in der Regel in Teströhrchen durchgeführt. Hierbei ist der gegen das Hapten gerichtete Antikörper an Partikel gebunden, die von einem Magneten angezogen werden können. In bevorzugter Weise handelt es sich hierbei um magnetisierbare Latexkügelchen, die Magnetit-Teilchen enthalten können, die von Polystyrol umhüllt sind, wobei der Durchmesser des Latexkügelchens bei bis zu 5 µm, bevorzugterweise bei 0,5 bis 2 µm liegt. Diese Antikörpermagnetpartikel werden mit der zu untersuchenden Flüssigkeit in Kontakt gebracht und können nach Reaktion mit Hilfe eines Magneten bequem präzipitiert werden.

Bei den erfindungsgemäßen Immunoassays handelt es sich in der Regel um kompetitive heterogene Immunoassays. Hierbei wird der gegen das Hapten gerichtete Antikörper an eine feste Phase gekoppelt, die mit der zu untersuchenden Probe in Kontakt gebracht wird. Zu der Probe wird weiterhin noch eine definierte Menge eines Haptens gegeben, das mit einer Anzeigekomponente verbunden ist. Wenn in der zu untersuchenden Probe kein Hapten vorhanden ist, können alle Antikörper mit Hapten reagieren, das eine Anzeigekomponente aufweist; das maximale Signal wird daher bei Abwesenheit des Analyten in der zu untersuchenden Probe auftreten.

Wenn die Probe eine höhere Konzentration an Hapten enthält, konkurriert das in der Probe vorhandene Hapten mit dem Hapten, das mit der Anzeigekomponente verbunden ist. Je mehr freies Hapten in der Probe vorhanden ist, desto weniger Hapten, das mit einer Anzeigekomponente verbunden ist, kann an die feste Phase binden und desto schwächer wird daher das Signal. Über entsprechende Eichkurven kann dann die tatsächliche Konzentration an Hapten in der zu untersuchenden Probe ermittelt werden.

Die erfindungsgemäßen Haptentracer-Antikörper-Komplexe eignen sich daher zur Verwendung in einem Immunoassay zum Nachweis eines Antigens bzw. Haptens, wobei es sich hierbei in der Regel um einen kompetitiven Immunoassay handelt. Bei den Immunoassays kann die Lumineszenz in Abhängigkeit von den jeweiligen Anzeigekomponenten nach an sich bekannten Methoden gemessen werden. Bei dem bevorzugt verwendeten Acridiniumacylsulfonamid kann die Messung der Lumineszenz beispielsweise dadurch erfolgen, daß die entsprechende Festphase mit einer alkalischen Wasserstoffperoxidlösung oder mit einer sauren Wasserstoffperoxidlösung mit unmittelbar nachfolgender pH-Erhöhung in Kontakt gebracht wird. Die dabei auftretende Lichtreaktion kann in bekannten Geräten, beispielsweise dem BeriLux® -Analyzer gemessen werden. Es hat sich gezeigt, daß die Handhabung, Empfindlichkeit und Präzision derartiger Lumineszenzimmunoassays für Haptene völlig vergleichbar ist mit der von entsprechenden Radioimmunoassays, wobei bei den erfindungsgemäßen Lumineszenzimmunoassays die Verwendung von radioaktiven Komponenten vermieden werden kann. Darüber hinaus sind die erfindungsgemäßen Lumineszenzimmunoassays für die Automation geeignet und können auch in Vollautomaten verwendet werden.

Die erfindungsgemäßen Haptentracer-Antikörper-Komplexe weisen eine Reihe von Vorteilen beim Einsatz in Immunoassays auf. Zunächst sind die Haptentracer-Antikörper-Komplexe einfach herzustellen. Die mit der Anzeigekomponente verbundenen Haptene werden in wäßriger Lösung mit dem Antikörper zusammengebracht. Dabei erfolgt eine spezifische Reaktion zwischen der Anzeigekomponente und der Bindungsregion des Antikörpers. Wenn monoklonale Antikörper gegen die Anzeigekomponente verwendet werden, ergibt sich auch die Möglichkeit, über die Wahl des Antikörpers das Signal zu beeinflussen.

Die erfindungsgemäßen Haptentracer-Antikörper-Komplexe haben eine definierte Einbaurate. Im Falle eines IgG-Antikörpers können zwei Moleküle Hapten-Anzeige-Komponente pro Molekül Anti-Anzeigekomponente-Antikörper gebunden werden. Im Falle eines Fab-Fragments verringert sich die Einbaurate auf ein Molekül Hapten-Anzeigekomponente pro Molekül Anti-Anzeigekomponente-Antikörperfragment. Durch Einsatz eines molaren Unterschusses an Hapten-Anzeigekomponente läßt sich die Einbaurate aber auch kontrollierbar verringern.

Durch das Vorhandensein des gegen die Anzeigekomponente gerichteten Antikörpers wird die Hydrophilie des Komplexes erhöht. Die hydrophoben Eigenschaften des Haptentracer-Antikörper-Komplexes sind aufgrund des hohen Proteinanteils und meist zusätzlich durch die direkte Bindung des in der Regel hydrophoben Anzeigeteiles an der spezifischen Bindungsregion des Anti-Anzeigekomponente-Antikörpers deutlich geringer als die des freien Haptentracers. Dies wirkt sich positiv auf die Wasserlöslichkeit des Tracers aus und führt in geringerem Maße zu unspezifischer Bindung. Durch die erfindungsgemäßen Komplexe kann also eine erhöhte Meßgenauigkeit erzielt werden.

Ein weiterer Vorteil der erfindungsgemäßen Komplexe ist, daß eine erhöhte Stabilität des Komplexes erzielt werden kann. Gerade bei Haptentracern, deren Anzeigekomponente hydrolyseempfindlich ist, zeigt sich bei längerer Aufbewahrung in wäßriger Lösung eine mehr oder weniger ausgeprägte Abnahme der Signalaktivität. Die Bindung der Anzeigekomponente an die spezifische Bindungsregion des Anti-Anzeigekomponente-Antikörpers ist in der Regel mit einem signifikanten Hydrolyseschutz verbunden.

Der einfach durchzuführende Einbau von zwei Anzeigekomponenten pro Antikörpermolekül bzw. einer Anzeigekomponente pro Fab-Fragment führt zu Haptentracer-Antikörper-Komplexen, die eine Signalaktivität besitzen.

Die vorliegende Erfindung wird durch die nachfolgenden Beispiele erläutert.

### Beispiel 1 (IgG; beschichtete Röhrchen)

### A) Herstellung eines T3 (= Triiodthyronin)-Haptentracers

Die Strukturformel des hergestellten Acridiniumacylsulfonamid-markierten Triiodthyronins ist in Abbildung 1a dargestellt.

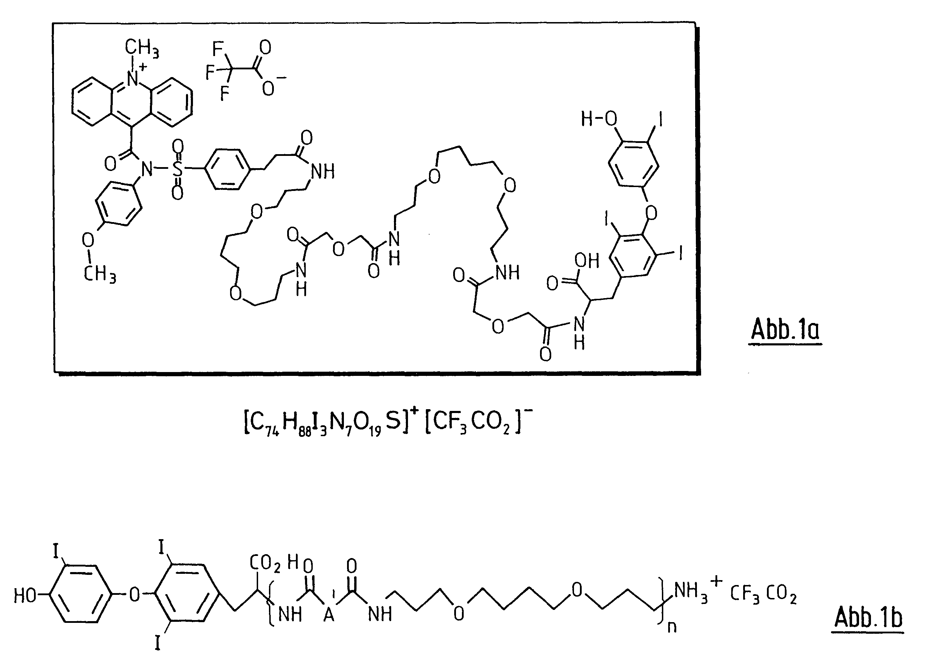

Man legt 220 mg (0,162 mmol) T3-Derivat, das in Abbildung 1b dargestellt ist, worin A' die Bedeutung -CH₂-O-CH₂- hat und n den Wert 2 aufweist (auch beschrieben in EP 0 442 372), in 3 ml trockenem N,N-Dimethylformamid (DMF) vor, gibt eine Lösung von 124 mg (0,162 mmol) BeriLux Label (mit N-Hydroxysuccinimidyl-Reaktivgruppe) in 2 ml DMF zu und rührt die Mischung 48 h bei Raumtemperatur. Das Lösungsmittel wird im Ölpumpenvakuum abdestilliert. Der Rückstand wird durch präparative Mitteldruckchromatographie (System Büchi) an einer Reversed Phase Säule [Stationäre Phase: LichroPrep C-18 (Fa. Merck); Mobile Phase: Methanol/Wasser = 70:30 + 0,1 Vol.% Trifluoressigsäure] gereinigt. Man isoliert 167 mg (52 %) gelb-oranges Pulver. [C₇₄H₈₉I₃N₇O₁₉S]⁺(1792) [CF₃CO₂]⁻ (113); MS: (FAB) 1792 (M⁺).

### B) Herstellung des T3-Haptentracer-Antikörper-Komplexes

### a) Anti-Anzeigekomponente-Antikörper:

Monoklonaler Maus-Antikörper (BW 90-9/016, Behringwerke AG, Marburg), gerichtet gegen das BeriLux-Label, gelöst in 0,5 M Natriumacetat, 0,5 M NaCl,, 50 mM Tris-HCl, pH 7,0, 0,01 % Natriumazid. Die Konzentration des Antikörpers betrug 11,5 mg/ml.

### b) Herstellung und Reinigung:

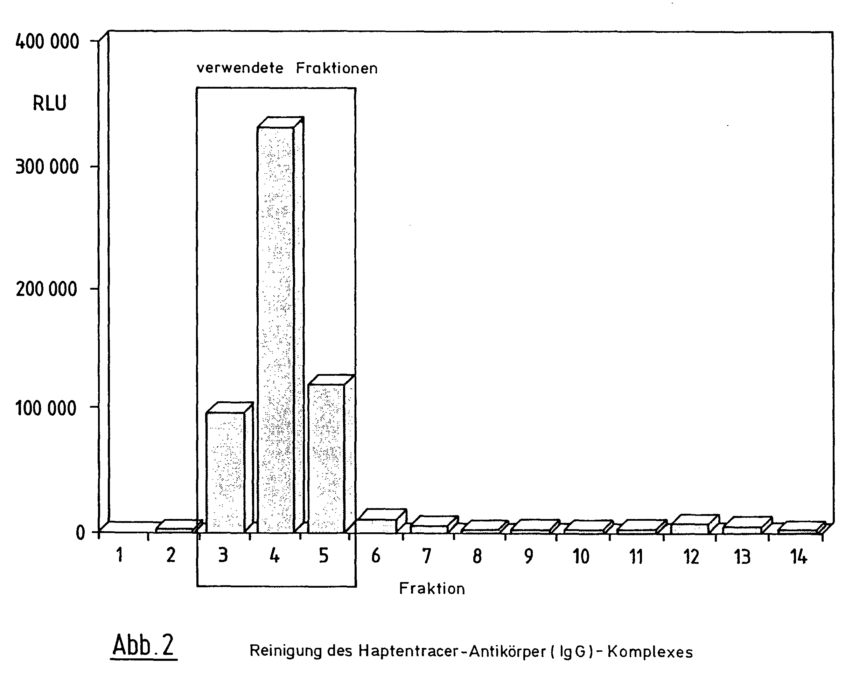

600 µl Inkubationspuffer (10 mM Phosphat, 8 g NaCl/l, 0,2 g KCl/l, pH 7,3), 8,7 µl Anti-Label-Antikörper-Lösung (siehe a), 60 µl Acetonitril und 2,5 µl T3-Haptentracer (A) (1 mg/ml Acetonitril) werden gemischt und 15 Minuten bei RT inkubiert. Die Reinigung erfolgte gelchromatographisch (PD-10 Sephadex G-25M; Code-No. 17-0851-01, Bettvolumen 9 ml, Fraktionsgröße 0,5 ml). Das Elutionsprofil ist in Abbildung 2 dargestellt.

### C) Verwendung des T3-Haptentracer-Antikörper-Komplexes in einem Lumineszenz-Immunoassay zur Bestimmung von FT3 (freier Anteil von Triiodthyronin)

### a) Festphase

Als Festphase dienten die beschichteten Röhrchen (coated tubes) des BeriLux FT3 Kits.

### b) Tracer

Der unter B) beschriebene Tracer wurde in einer Konzentration von 150 ng/ml BeriLux FT3-Tracerpuffer aufbewahrt.

### c) FT3-Standards

Es wurden die FT3-Standards mit einer Konzentration im Bereich 0 bis 50 pg/ml Serummatrix eingesetzt.

### d) Assaydurchführung

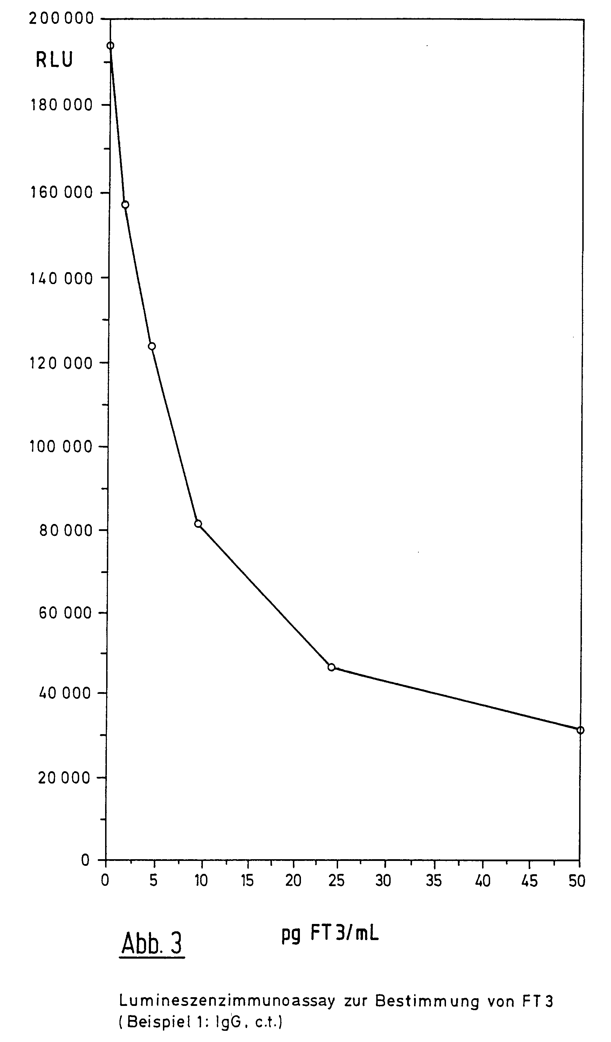

100 µl Probe und 200 µl BeriLux FT3-Inkubationspuffer wurden in BeriLux FT3 coated tubes pipettiert. Die Röhrchen wurden 30 Minuten bei RT (Raumtemperatur) geschüttelt (300 Upm). Nach zweimaligem Waschen mit je 0,5 ml PBS-Puffer wurden 200 µl Tracer zupipettiert und weitere 5 Minuten bei RT geschüttelt. Nach dreimaligem Waschen mit je 0,5 ml PBS-Puffer wurden die Röhrchen im BeriLux Analyzer gemessen. Die Ergebnisse sind in Abbildung 3 dargestellt (RLU bedeutet "Relative Light Units")

### Beispiel 2 (IgG; Magnetpartikel)

### A) und B)

Wie in Beispiel 1.

### C) Verwendung des T3-Haptentracer-Antikörper-Komplexes in einem Lumineszenz-Immunoassay zur Bestimmung von FT3

### a) Festphase:

Magnetpartikel der Fa. Rhone-Poulenc (Artikel-Nr. EM1-100/20) wurden mit einem monoklonalen Anti-T3-Antikörper (BeriLux FT3-Antikörper) nach der Carbodiimid-Methode [G. Wendlberger, P. Stelzel, Synthese von Peptiden, Teil II (Methoden Org. Chem.) Houben-Weyl, 4th ed. 1952, Bd. XV/2, 1974] beschichtet. Die Beschichtungskonzentration betrug 0,25 mg Antikörper pro ml 10 %ige Magnetpartikelsuspension. Die gebrauchsfertige Suspension hatte eine Magnetpartikel-Konzentration von 2,5 mg pro ml Lagerpuffer (10,36 g CHES, 0,5 g Natriumazid, 1 g Rinder-IgG pro Liter, pH 8,0).

### b) und c) Tracer und FT3-Standards

Wie in Beispiel 1.

### d) Assaydurchführung

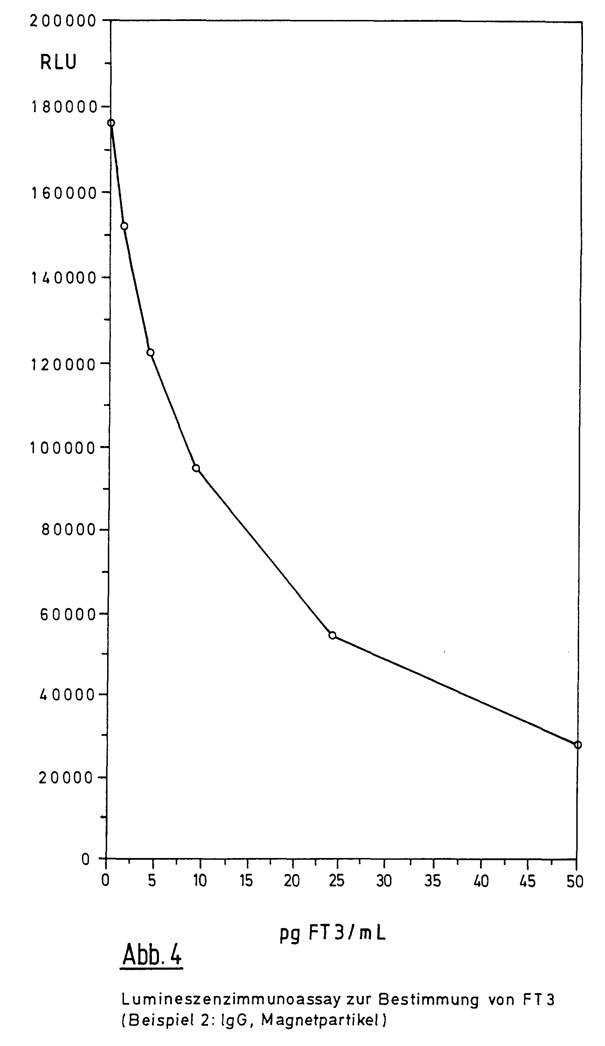

20 µl beschichtete Magnetpartikel (a), 100 µl Probe und 200 µl BeriLux FT3-Inkubationspuffer wurden in einem Polystyrolröhrchen 30 Minuten bei 37 °C inkubiert. Nach zweimaligem Waschen der Festphase mit je 0,5 ml PBS-Puffer wurden 200 µl Tracer zugegeben und 5 Minuten bei RT inkubiert. Nach dreimaligem Waschen mit je 0,5 ml PBS-Puffer wurde das Röhrchen im BeriLux Analyzer gemessen. Die Ergebnisse sind in Abbildung 4 dargestellt.

### Beispiel 3 (Fab; beschichtete Röhrchen)

### A) Herstellung des T3-Haptentracers

Wie in Beispiel 1.

### B) Herstellung des Komplexes aus T3-Haptentracer und Antikörper-Fab-Fragment

### a) Anti-Anzeigekomponente-Antikörper (Fab-Fragment)

Durch Papain-Spaltung gewonnenes Fab-Fragment des monoklonalen Maus-Antikörpers (BW 90-9/016, Behringwerke AG, Marburg), gerichtet gegen das BeriLux-Label, gelöst in 0,5 M Natriumacetat, 0,5 M NaCl, 50 mM Tris-HCI, pH 7,0, 0,01 % Natriumazid. Die Konzentration des Fab-Fragments betrug 1 mg/ml.

### b) Herstellung und Reinigung:

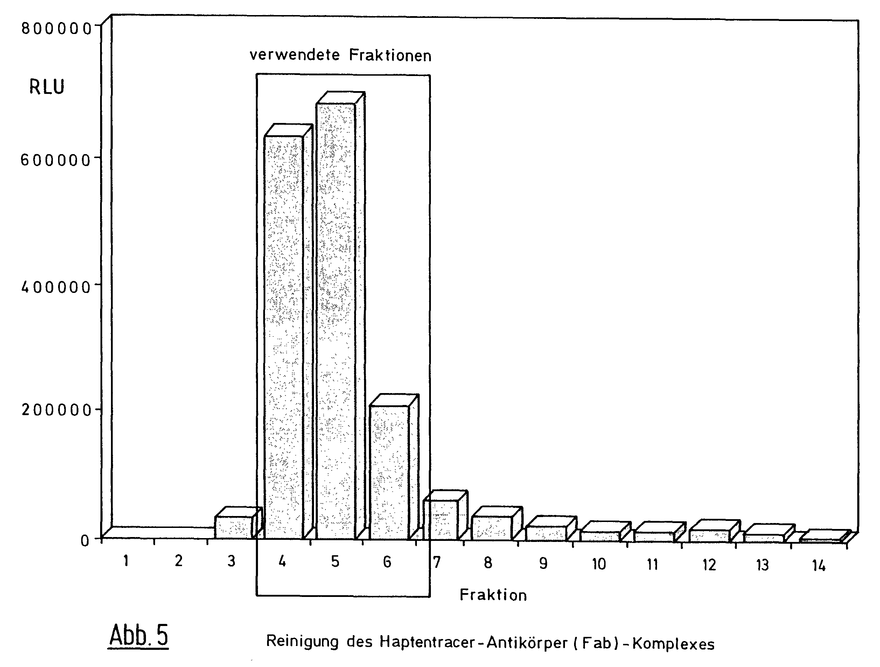

500 µl Inkubationspuffer (10 mM Phosphat, 8 g NaCl/l, 0,2 g KCl/l, pH 7,3), 100 µl Anti-Label-Antikörper (Fab)-Lösung (siehe a), 60 µl Acetonitril und 7,6 µl T3-Haptentracer (A) (1 mg/ ml Acetonitril) werden gemischt und 15 Minuten bei RT inkubiert. Die Reinigung erfolgte gelchromatographisch (PD-10 Sephadex G-25M; Code-No. 17-0851-01, Bettvolumen 9 ml, Fraktionsgröße 0,5 ml). Das Elutionsprofil ist in Abbildung 5 dargestellt.

### C) Verwendung des T3-Haptentracer-Antikörper-Fab-Fragment-Komplexes in einem Lumineszenz-Immunoassay zur Bestimmung von FT3

### a)-d) Festphase, Tracer, FT3-Standards und Assaydurchführung

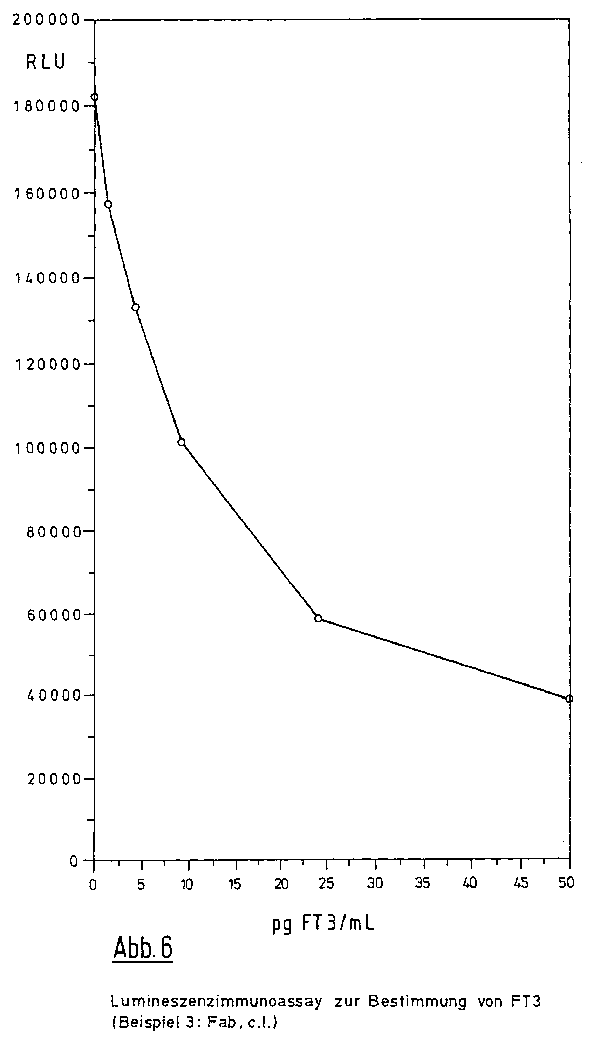

Wie in Beispiel 1, jedoch mit einer Tracerkonzentration von 50 ng/ml. Die Standardkurve ist in Abbildung 6 dargestellt.

### Beispiel 4 (Fab; Magnetpartikel)

### A) und B)

Wie in Beispiel 3.

### C) Verwendung des T3-Haptentracer-Antikörper-Fab-Fragment-Komplexes in einem Lumineszenz-Immunoassay zur Bestimmung von FT3

### a)-d) Festphase, Tracer, FT3-Standards und Assaydurchführung

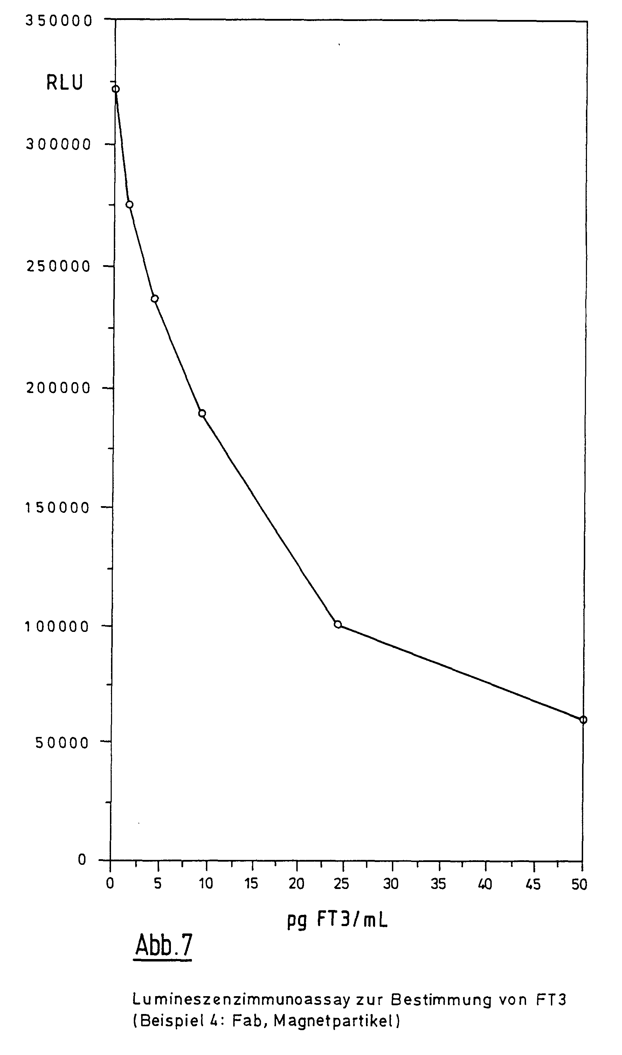

Wie in Beispiel 2. Die Standardkurve ist in Abbildung 7 dargestellt.

### Beispiel 5 (Signalstabilität des freien Haptentracers)

Um die Instabilität einer Haptentracer-Lösung (Lösung, die den freien, also nicht durch den Anti-Label-Antikörper komplexierten Haptentracer enthält) zu zeigen, wurden 0,3 mg T3-Haptentracer, wie in Abbildung 1a dargestellt, in 0,3 ml Acetonitril klar gelöst (1 mg/ml). Diese Lösung wurde mit Tracerpuffer (100 mM Phosphatpuffer, pH 6,3 mit 8,8 g NaCl/l, 1 g Mergal K9N/l, 2 g Rinder-IgG/l) auf eine Konzentration von 10 ng T3-Haptentracer pro ml verdünnt (= Stammlösung).

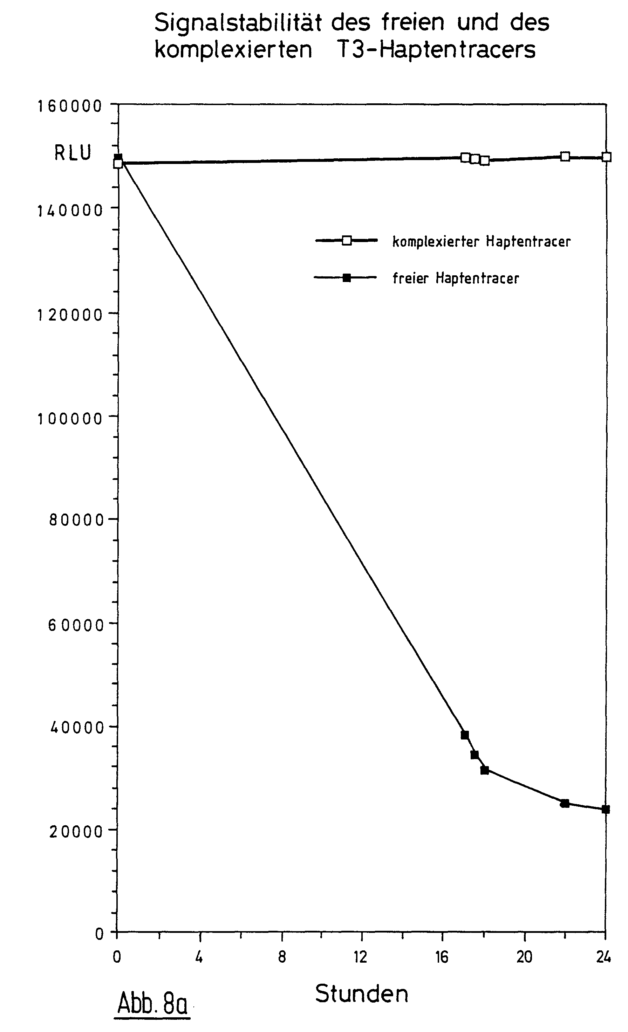

Nach verschiedenen Zeiten wurden aus dieser Stammlösung Proben entnommen und in einem Luminometer gemessen. Die Abbildung 8a zeigt deutlich, daß trotz Vorhandensein von Schutzprotein in Form von Immunglobulin die Signalaktivität von Probe zu Probe rasch abfiel. Das Signal des komplexierten Haptentracers bleibt hingegen konstant.

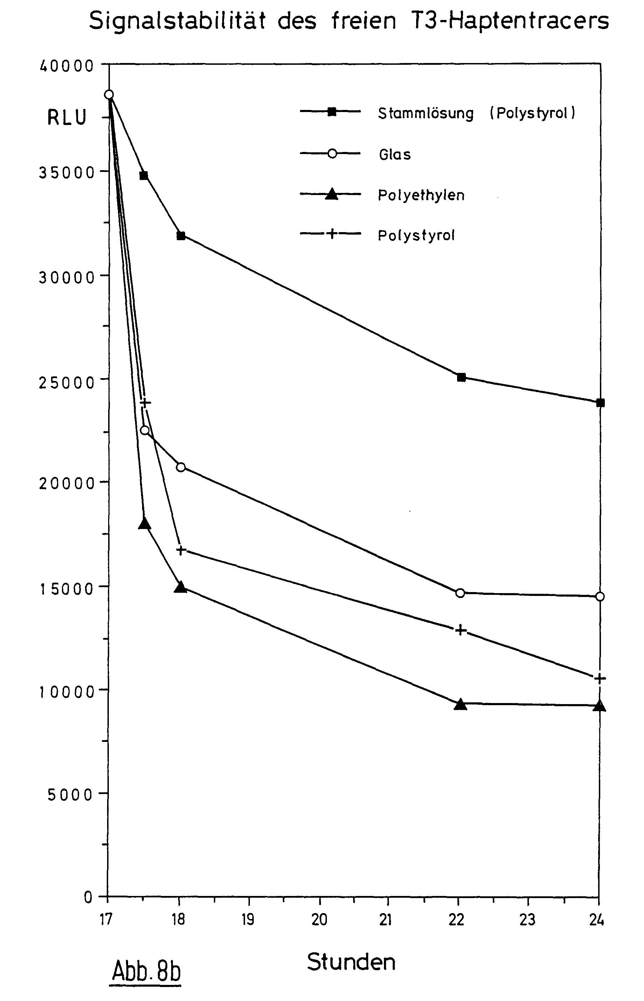

Nach 17 Stunden wurde ein Teil der Stammlösung zu gleichen Teilen in andere (gleich dimensionierte) Gefäße (aus Glas, Polystyrol bzw. Polyethylen) umgefüllt. Wie aus der Abbildung 8b ersichtlich, tritt daraufhin ein erneuter starker Abfall der Signalaktivität ein, der zudem stark vom Material des verwendeten Gefäßes abhängt. Die Zeitangabe auf der Abszisse bezieht sich auf das Ansetzen der Stammlösung.

## Patentansprüche

1. Haptentracer-Komplex **dadurch gekennzeichnet, daß** er einerseits ein Hapten aufweist, das direkt oder über'eine Abstandsgruppe mit einer Anzeigekomponente verbunden ist und andererseits einen Antikörper aufweist, der spezifisch an die Anzeigekomponente binden kann.

2. Komplex nach Anspruch 1 **dadurch gekennzeichnet, daß** die Anzeigekomponente eine zur Chemolumineszenz fähige Gruppe ist.

3. Komplex nach Anspruch 2, **dadurch gekennzeichnet, daß** die Anzeigekomponente eine Verbindung ist, die ausgewählt ist aus der Gruppe umfassend Acridiniumacylsulfonamid, Acridiniumester, Luminol, Isoluminol, Dioxetan, Oxalsäurester oder Oxalsäureamid oder ein Derivat einer dieser Verbindungen.

4. Komplex nach Anspruch 1 **dadurch gekennzeichnet, daß** die Anzeigekomponente eine zur Fluoreszenz fähige Gruppe ist.

5. Komplex nach Anspruch 1 **dadurch gekennzeichnet, daß** die Anzeigekomponente eine zur Biolumineszenz fähige Gruppe ist.

6. Komplex nach Anspruch 1 **dadurch gekennzeichnet, daß** die Anzeigekomponente eine zur Elektrolumineszenz fähige Gruppe ist.

7. Komplex nach Anspruch 1 **dadurch gekennzeichnet, daß** die Anzeigekomponente eine zur Phosphoreszenz fähige Gruppe ist.

8. Komplex nach einem der Ansprüche 1 bis 7 **dadurch gekennzeichnet, daß** der gegen die Anzeigekomponente gerichtete Antikörper ein monoklonaler Antikörper, ein polyklonaler Antikörper, ein Antikörperfragment, ein chemisch modifizierter Antikörper oder ein chemisch modifiziertes Antikörperfragment ist.

9. Verfahren zur Herstellung eines Komplexes nach einem der Ansprüche 1 bis 8, **dadurch gekennzeichnet, daß** man den gegen die Anzeigekomponente gerichteten Antikörper mit dem Hapten, das mit der Anzeigekomponente verbunden ist, in wäßriger Lösung zusammenbringt, wobei die wäßrige Lösung zur Löslichkeitsverbesserung des mit der Anzeigekomponente verbundenen Haptens auch organische Lösungsmittel, insbesondere Acetonitril, Dimethylformamid oder Dimethylsulfoxid enthalten kann.

10. Testkit zur Durchführung eines Immunoassays zum Nachweis eines Antigens und/oder Haptens **dadurch gekennzeichnet, daß** das Testkit einen Komplex nach einem der Ansprüche 1 bis 8 aufweist.

11. Testkit nach Anspruch 10 **dadurch gekennzeichnet, daß** es ein Testkit zur Durchführung eines Lumineszenzimmunoassays ist.

12. Testkit nach einem der Ansprüche 10 oder 11 **dadurch gekennzeichnet, daß** es zur Durchführung eines kompetitiven Immunoassays geeignet ist.

13. Testkit nach Anspruch 10 bis 12 **dadurch gekennzeichnet, daß** es weiterhin magnetisierbare Partikel aufweist, an die die gegen das Hapten gerichteten Antikörper gebunden sind.

14. Verwendung eines Komplexes nach einem der Ansprüche 1 bis 8 in einem Immunoassay zum Nachweis eines Antigens und/oder Haptens.

15. Verwendung nach Anspruch 14 **dadurch gekennzeichnet, daß** es sich um einen kompetitiven Immunoassay handelt.

## Claims

1. A hapten tracer complex which, on the one hand, contains a hapten which is linked directly or via a spacer group to an indicator component and, on the other hand, contains an antibody which can bind specifically to the indicator component.

2. A complex as claimed in claim 1, wherein the indicator component is a group capable of chemiluminescence.

3. A complex as claimed in claim 2, wherein the indicator component is a compound which is selected from the group comprising acridinium acylsulfonamide, acridinium ester, luminol, isoluminol, dioxetane, oxalic acid ester or oxalamide or a derivative of one of these compounds.

4. A complex as claimed in claim 1, wherein the indicator component is a group capable of fluorescence.

5. A complex as claimed in claim 1, wherein the indicator component is a group capable of bioluminescence.

6. A complex as claimed in claim 1, wherein the indicator component is a group capable of electroluminescence.

7. A complex as claimed in claim 1, wherein the indicator component is a group capable of phosphorescence.

8. A complex as claimed in one of claims 1 to 7, wherein the antibody directed against the indicator component is a monoclonal antibody, a polyclonal antibody, an antibody fragment, a chemically modified antibody or a chemically modified antibody fragment.

9. A process for the preparation of a complex as claimed in one of claims 1 to 8, wherein the antibody directed against the indicator component is brought together in aqueous solution with the hapten which is linked to the indicator component, it being possible, to improve the solubility of the hapten linked to the indicator component, for the aqueous solution also to contain organic solvents, in particular acetonitrile, dimethylformamide or dimethyl sulfoxide.

10. A test kit for carrying out an immunoassay for the detection of an antigen and/or hapten, wherein the test kit contains a complex as claimed in one of claims 1 to 8.

11. A test kit as claimed in claim 10, which is a test kit for carrying out a luminescence immunoassay.

12. A test kit as claimed in one of claims 10 or 11, wherein it is suitable for carrying out a competitive immunoassay.

13. A test kit as claimed in claims 10 to 12, wherein it furthermore contains magnetizable particles to which the antibodies directed against the hapten are bound.

14. The use of a complex as claimed in one of claims 1 to 8 in an immunoassay for the detection of an antigen and/or hapten.

15. The use as claimed in claim 14, wherein a competitive immunoassay is concerned.

## Revendications

1. Complexe d'haptène-marqueur, **caractérisé en ce qu'**il comporte d'une part un haptène qui est lié directement ou par l'intermédiaire d'un groupe espaceur à un composant indicateur, et comporte d'autre part un anticorps qui peut se fixer spécifiquement au composant indicateur.

2. Complexe selon la revendication 1, **caractérisé en ce que** le composant indicateur est un groupe apte à la chimiluminescence.

3. Complexe selon la revendication 2, **caractérisé en ce que** le composant indicateur est un composé qui est choisi dans l'ensemble constitué par l'acylsulfonamidure d'acridinium, un ester d'acridinium, le luminol, l'isoluminol, le dioxétanne, un ester d'acide oxalique ou l'oxalamide, ou un dérivé d'un de ces composés.

4. Complexe selon la revendication 1, **caractérisé en ce que** le composant indicateur est un groupe apte à la fluorescence.

5. Complexe selon la revendication 1, **caractérisé en ce que** le composant indicateur est un groupe apte à la bioluminescence.

6. Complexe selon la revendication 1, **caractérisé en ce que** le composant indicateur est un groupe apte à l'électroluminescence.

7. Complexe selon la revendication 1, **caractérisé en ce que** le composant indicateur est un groupe apte à la phosphorescence.

8. Complexe selon l'une quelconque des revendications 1 à 7, **caractérisé en ce que** l'anticorps dirigé contre le composant indicateur est un anticorps monoclonal, un anticorps polyclonal, un fragment d'anticorps, un anticorps modifié chimiquement ou un fragment d'anticorps modifié chimiquement.

9. Procédé pour la préparation d'un complexe selon l'une quelconque des revendications 1 à 8, **caractérisé en ce qu'**on met l'anticorps dirigé contre le composant indicateur en contact avec l'haptène qui est lié au composant indicateur, en solution aqueuse, la solution aqueuse pouvant également, pour l'amélioration de la solubilité de l'haptène lié au composant indicateur, contenir des solvants organiques, en particulier de l'acétonitrile, du diméthylformamide ou du diméthylsulfoxyde.

10. , Nécessaire d'essai pour l'exécution d'un essai immunologique destiné à la détection d'un antigène et/ou d'un haptène, **caractérisé en ce que** le nécessaire d'essai comporte un complexe selon l'une quelconque des revendications 1 à 8.

11. Nécessaire d'essai selon la revendication 10, **caractérisé en ce qu'**il est un nécessaire d'essai pour l'exécution d'un essai immunologique par luminescence.

12. Nécessaire d'essai selon la revendication 10 ou 11, **caractérisé en ce qu'**il est approprié à l'exécution d'un essai immunologique compétitif.

13. Nécessaire d'essai selon les revendications 10 à 12, **caractérisé en ce qu'**il comporte en outre des particules magnétisables, sur lesquelles sont fixés des anticorps dirigés contre l'haptène.

14. Utilisation d'un complexe selon l'une quelconque des revendications 1 à 8, dans un essai immunologique pour la détection d'un antigène et/ou d'un haptène.

15. Utilisation selon la revendication 14, **caractérisée en ce qu'**il s'agit d'un essai immunologique compétitif.
